(19) **Europäisches Patentamt**

**European Patent Office**

**Office européen des brevets**

(11) **EP 1 033 360 B1**

(12) **EUROPÄISCHE PATENTSCHRIFT**

(45) Veröffentlichungstag und Bekanntmachung des Hinweises auf die Patenterteilung:
**17.09.2003 Patentblatt 2003/38**

(51) Int Cl.$^7$: **C07C 67/465**, C07C 69/01

(21) Anmeldenummer: **00103784.5**

(22) Anmeldetag: **23.02.2000**

(54) **Verfahren zur Herstellung von Vinylestern aus Butenoligomeren**

Process for preparing vinylesters from butene oligomers

Procédé de préparation d'esters vinyliques à partir d'oligomers de butène

(84) Benannte Vertragsstaaten:
**BE DE ES FR GB IT NL SE**

(30) Priorität: **26.02.1999 DE 19908320**

(43) Veröffentlichungstag der Anmeldung:
**06.09.2000 Patentblatt 2000/36**

(73) Patentinhaber: **Oxeno Olefinchemie GmbH**
**45764 Marl (DE)**

(72) Erfinder:
• **Wiese, Klaus-Diether, Dr.**
  **45721 Haltern (DE)**
• **Olbrich, Paul, Dr.**
  **45721 Haltern (DE)**
• **Gabriel, Jürgen, Dr.**
  **46282 Dorsten (DE)**

(56) Entgegenhaltungen:
**EP-A- 0 820 973**      **WO-A-92/13818**
**WO-A-93/22353**

**Beschreibung**

Die Erfindung betrifft ein Verfahren zur Herstellung von Vinylestern auf der Basis von Butenoligomeren, insbesondere auf Basis von Dibuten und Tributen, sowie die Verwendung dieser Vinylester.

Vinylester tertiärer Carbonsäuren haben seit langem einen festen Platz in der Technik als Comonomere, insbesondere als innere Weichmacher fiir die Herstellung von umweltfreundlichen wasserdispergierbaren Lacken und Farben auf Basis von Vinylacetat. Neben der Wirkung als Weichmacher verleihen sie den Copolymeren weitere vorteilhafte Eigenschaften wie hohe Verseifungsstabilität, die die Copolymere für den Einsatz unter rauhen Bedingungen geeignet machen. Erwähnt seien beispielhaft Außenanstriche und Wärmeschutzputze von Gebäuden.

Die weichmachenden Eigenschaften der Vinylester, insbesondere der tertiärer Carbonsäuren hängen von ihrer Kettenlänge und von Art und Stellung der Verzweigungen ab. Ein Maß für die innere Weichmachung von Copolymerisaten ist der Glaspunkt des entsprechenden Homopolymerisats. Ein Vergleich der weichmachenden Eigenschaften von Vinylestern verschiedener Kettenlängen mittels des Glaspunktes $T_g$ des jeweiligen Homopolymerisats zeigt dessen Abhängigkeit von der Molmasse bzw. vom Verzweigungsgrad:

| Kettenlänge der Carbonsäure | Linearer Vinylester | Glaspunkt [°C] | tertiärer Vinylester | Glaspunkt [°C] |
|---|---|---|---|---|
| $C_2$-Vinylester | Vinylacetat | +38(33)* | | |
| $C_3$-Vinylester | Vinylpropionat - | 7 (-7)* | | |
| $C_4$-Vinylester | Vinylbutyrat | - 5 | | |
| $C_5$-Vinylester | | -15*** | 2,2-Dimethyl-Propansäure | 86 (70)* |
| $C_6$ Vinylester | Vinylhexanoat | -20 | 2,2-Dimethyl-Butansäure | 41** |
| $C_{10}$-Vinylester | Vinyldecanoat | - 60 | | |
| $C_{12}$-Vinylester | Vinyllaurat | - 75 (-53)* | | |
| (Encyclopedia of Polymer Science and Engineering, Vol. 17, S. 439 (1989), J. Wiley & Sons. Inc.), (* Ullmann's Encyclopedia of Industrial Chemistry, Vol. A22, S. 2, 5. Ed. (1993), Verlag Chemie), (** C.E.L. Feeder, Surface Coatings Austral. 228 1985), 8, S. 11-16). (*** eigene Messung) | | | | |

Diese Werte sind nur als Vergleichsdaten untereinander geeignet, da sie mit der Herstellungsmethode der Prüflinge und mit der Prüfmethode variieren können. Es zeigt sich jedoch, daß sich die weichmachenden Eigenschaften mit zunehmender Kettenlänge des Vinylesters (bei linearen Ketten bis zu einem Alkylrest mit 12 Kohlenstoffatomen) verbessern. Insbesondere haben die Vinylester von linearen Carbonsäuren sehr gute weichmachende Eigenschaften, sie sind jedoch für viele Anwendungen weniger geeignet, da sie leicht verseifbar sind.

Vielseitiger einsetzbar sind hingegen die Vinylester tertiärer Carbonsäuren, da sie sehr verseifungs-, temperatur- und oxidationsstabil sind. Allerdings erniedrigt die tertiäre Verzweigung die weichmachende Wirkung drastisch, und weitere Verzweigungen in der Kette sorgen für weitere Verschlechterung, wie folgende Beispiele von Homopolymerisaten von Vinylestern tertiärer $C_9$-Carbonsäuren zeigen:

| Tertiäre $C_9$-Carbonsäuren | Glaspunkt [°C] |
|---|---|
| 2,3-Dimethyl-2-Isopropyl-Butansäure | 119 |
| 2-Ethyl-2,3, 3-Trimethyl-Butansäure | 115 |
| 2,2,3,3-Tetramethyl-Pentansäure | 91 |
| (VeoVa®9, Shell) | 70 (60)* |
| 2,4,4-Tetramethyl-Pentansäure | 55 |
| 2,2,4-Trimethyl-Hexansäure | 10 |
| (H. P. H. Scholten, J. Vermeulen, W. J. van Westrenen, Recent Development in Latices based on Vinyl Esters of branched Monocarboxylic Acids, 7. International Conference on "Water-Borne Coatings" 26.-28.10.98, Penta Hotel, London), (* W. Lau, VeoVa®, Vinyl Ester Monomer Polymers DotCom Magazine, Vol. 2., Nr. 2, Feb. 1996) | |

Technisch werden heute vorwiegend Vinylester, hergestellt aus einem Isomerengemisch tertiärer $C_{10}$-Carbonsäuren, die Homopolymerisate mit einem Glaspunkt von - 3 °C bilden, eingesetzt Dieses Gemisch ist für den Einsatz z. B. zur inneren Weichmachung von Polyvinylacetat unter gleichzeitiger Erhöhung der Verseifungsstabilität sehr geeignet und gefragt.

Die zur Herstellung der Vinylester eingesetzte $C_{10}$-Carbonsaure wird ihrerseits durch Anlagerung von Kohlenmonoxid und Wasser an Tripropen unter Druck und Katalyse mit extrem sauren Katalysatoren (Hydrocarboxylierung, insbesondere sogenannte KOCH-Reaktion) hergestellt.

Tripropen schließlich, ein Gemisch isomerer $C_9$-Olefine, wird im Gemisch mit anderen Olefin-Fraktionen ($C_6$-, $C_{12}$-, $C_{15}$-Olefine) durch sauer katalysierte Oligomerisierung von Propen erhalten. Als Katalysatoren kommen hier z. B. saure Zeolithe oder Phosphorsäure auf einem festen Träger in Frage.

Nachteilig bei den Herstellverfahren von Vinylestern von $C_{10}$-Carbonsäuren ist, daß mit Propen ein vergleichsweise teurer Rohstoff benötigt wird. Bei dessen sauer katalysierten Oligomerisierung ist weiter wegen Nebenproduktbildung mit deutlichen Rohstoffverlusten zu rechnen. Schließlich ist darauf hinzuweisen, daß schon im Tripropen-Anteil der Oligomerisate die Isomerenanzahl so hoch ist, daß selbst eine analytische Kontrolle schwierig ist. Bei der Umsetzung zu Carbonsäuren schließlich entsteht eine so hohe Anzahl von Isomeren, das ein Produktgemisch mit schwer zu definierenden Eigenschaften resultiert.

Vinylester von tertiären Carbonsäuren mit mehr als 10 C-Atomen sind teilweise untersucht (z. B. WO 93/22353). Sie haben durchaus weichmachende Wirkung, wie auf Grund ihrer längeren Kohlenstoffkette erwartet werden kann. Aber einerseits sind die notwendigen Rohstoffe häufig nicht preiswert genug verfügbar, andererseits steigt mit wachsender Kettenlänge auch die sogenannte Unverträglichkeit im Copolymerisat.

Die Vinylester von tertiären Carbonsäuren mit weniger als 10 C-Atomen sind ebenfalls teilweise bekannt und auf ihre Eignung als Weichmacher untersucht worden. So haben die Vinylester auf Basis von Pivalinsäure (einer tertiären $C_5$-Säure) und auf Basis von tertiären $C_9$-Säuren (VeoVa®9) eine gewisse technische Bedeutung, in beiden Fällen handelt es sich aber im Vergleich zu Vinylacetat um hartmachende Comonomere.

Es wäre daher wünschenswert, andere Rohstoffquellen als Propen bzw. dessen Oligomere zur Herstellung von Vinylestern zu erschließen, die gleiche oder bessere weichmachende Eigenschaften wie die Vinylester z. B. der $C_{10}$-Carbonsäuren auf Basis von Tripropen aufweisen.

Überraschenderweise wurde gefunden, daß tertiäre Carbonsäuren, die aus Butenoligomeren hergestellt wurden, hervorragend als Weichmacher geeignet sind.

Gegenstand der vorliegenden Erfindung ist daher ein Verfahren zur Herstellung von Vinylestern, wobei

a) Butene oligomerisiert,
b) die Butenoligomeren aus dem Oligomerisat abgetrennt,
c) die Butenoligomeren zu um ein Kohlenstoffatom längeren Carbonsäuren umgesetzt und
d) die Carbonsäuren zu den entsprechenden Vinylestern umgesetzt werden.

Die nach dem erfindungsgemäßen Verfahren hergestellten Vinylester eignen sich hervorragend als Comonomere für die Herstellung von innerlich weichgerriachten Polymeren. Beispielhaft erwähnt seien die Copolymerisation des $C_9$-Carbonsäurevinylesters mit Vinylchlorid, oder die Copolymerisation mit Vinylacetat. Auch Terpolymere mit Acrylaten sind ein potentielles Einsatzgebiet. Der Anteil an erfindungsgemäß hergestelltem Vinylester kann dabei in weiten Grenzen variieren, je nach den gewünschten Eigenschaften. Auch ihr Einsatz als Homopolymerisat ist eingeschlossen, wenn z. B. besonders weiche Filme hergestellt werden sollen.

Im erfindungsgemäßen Verfahren werden zunächst Butene oligomerisiert, wobei vorwiegend Dibuten ($C_8$-Olefin) entsteht. Daneben bilden sich Tributen ($C_{12}$-Olefin) und Tetrabuten ($C_{16}$-Olefin) durch Trimerisierung bzw. Tetramerisierung von Buten. Hier können alle technischen $C_4$-Olefin-haltigen Ströme als Rohstoff eingesetzt werden, wie z. B. Crack-$C_4$, $C_4$-Olefine aus Fischer-Tropsch-Verfahren, $C_4$-Olefine aus Dehydrierung von Butan oder aus sonstigen technischen Prozessen.

Im erfindungsgemäßen Verfahren können somit Di-, Tri, Tetrabuten oder noch höhere Oligomere als Butenoligomere eingesetzt werden. Die Abtrennung der Butenoligomeren aus dem Oligomerisat kann technisch einfach und mit hoher Reinheit durch Destillation erfolgen.

Das Verfahren der vorliegenden Erfindung kann sowohl zur Herstellung von $C_9$-Carbonsäurevinylestern aus Dibuten bzw. aus der entsprechenden $C_9$-Carbonsäure als auch zur Herstellung von $C_{13}$-Carbonsäurevinylestern aus Tributen bzw. aus der entsprechenden $C_{13}$-Carbonsäure eingesetzt werden.

Zur Gewinnung von wenig verzweigten Vinylestern müssen selbstverständlich weitgehend lineare $C_4$-Ströme, d.h. solche mit einem hohen Anteil an n-Butenen zur Oligomerisierung eingesetzt werden.

Üblicherweise wird aus dem rohen Crack-$C_4$ zunächst Butadien durch Extraktion entfernt oder durch Selektivhydrierung in lineare Butene überführt. Die Selektivhydrierung ist im vorliegenden Fall nicht zwingend notwendig, aber besonders günstig, da es den Anteil der n-Butene für die Oligomerisierung erheblich erhöht. In beiden Fällen wird

ein butadienfreier C$_4$-Schnitt erhalten, das Raffinat I. Im nächsten Schritt kann Isobuten aus dem C$_4$-Strom z. B. durch Herstellung von Methyl-tert.-Butylether (MTBE) durch Umsetzung mit Methanol entfernt werden. MTBE ist eine gefragte Kraftstoffkomponente. Andere Möglichkeiten sind die Umsetzung des Isobutens aus dem Raffinat I mit Wasser zu TBA (tertiär-Butanol) oder die sauer katalysierte Oligomerisierung des Isobutens zu Diisobuten. Der jetzt isobutenfreie C$_4$-Schnitt, das Raffinat II, enthält wie gewünscht nur noch 1-Buten und 2-Buten. Optional kann auch noch 1-Buten destillativ gewonnen werden, der 1-Buten-freie Schnitt wird dann als Raffinat III bezeichnet.

Eine einfache destillative Abtrennung des Isobutens mit nachfolgender Weiterverarbeitung ist aus einem C$_4$-Schnitt normalerweise nicht möglich, da 1-Buten und Isobuten fast den gleichen Siedepunkt aufweisen. Die destillative Trennung von 2-Buten und Isobuten ist jedoch möglich. Überführt man daher 1-Buten durch Hydroisomerisierung in 2-Buten, ist die einfache destillative Abtrennung des Isobutens ein gangbarer Weg, um zu C$_4$-Strömen zu gelangen, die nur noch lineare Butene enthalten.

Für die Oligomerisierung von Buten zum Dibuten wird bevorzugt Raffinat II oder III eingesetzt. Auch andere technische C$_4$-Ströme sind einsetzbar, wenn sie außer den linearen Butenen keine weiteren ungesättigten Verbindungen enthalten. Der besonders bevorzugte Einsatzstoff für die Butenoligomerisierung ist n-Buten, da die hieraus hergestellten Vinylester tertiärer Carbonsäuren, wie durch die Beispiele belegt, die besseren Weichmachereigenschaften aufweisen.

Spielt allerdings die weichmachende Wirkung der Vinylester eine untergeordnete Rolle oder ist sogar eine hartmachende Wirkung erwünscht, sind isobutenhaltige C$_4$-Ströme einsetzbar; ein bevorzugter Einsatzstoffist dann Raffinat I.

Durch das erfindungsgemäße Verfahren können mit technisch vorhandenen C$_4$-Strömen, die bisher häufig chemisch gar nicht genutzt wurden, wirtschaftliche Rohstoffe zur Herstellung von Vinylestern eingesetzt werden.

Die Oligomerisierung Buten-haltiger C$_4$-Ströme zu C$_8$-, C$_{12}$- und höhere Olefinen enthaltenden Gemischen ist grundsätzlich bekannt. Im Prinzip gibt es drei Verfahrensvarianten:

Lange bekannt ist die Oligomerisierung an sauren Katalysatoren, technisch eingesetzt werden z. B. Zeolithe oder Phosphorsäure auf Trägern. Hierbei werden Isomerengemische von verzweigten Olefinen erhalten. Selbst unter optimierten Bedingungen bleiben Dimethylhexene das Hauptprodukt (WO 92/13818).

Wie in den Beispielen gezeigt, können die dabei abgetrennten C$_8$-Olefine zu den entsprechenden tertiären Carbonsäuren und ihren Vinylestern verarbeitet werden. Es werden jedoch nur Homopolymere mit einem hohen Glaspunkt, und somit schlecht weichmachende oder sogar hartmachende Comonomere erhalten.

Ein ebenfalls weltweit ausgeübtes Verfahren ist die Oligomerisiserung mit löslichen Nickel-Komplexen, bekannt als DIMERSOL-Verfahren (s. Yves Chauvin, Helene Olivier; in "Applied Homogeneous Catalysis with Organometallic Compounds"; edited by Boy Cornils, Wolfgang A. Herrmann; Verlag Chemie, 1996, 258-268). Die aus der C$_8$-Fraktion hergestellten Vinylester der C$_9$-Carbonsäuren haben eine deutlich bessere weichmachende Wirkung wie die nach dem vorgenannten Verfahren erzeugten Vinylester (siehe Beispiele).

Schließlich ist noch die Oligomerisierung an Nickel-Festbett-Katalysatoren nach dem Verfahren der OXENO GmbH zu erwähnen. Das Verfahren hat in die Literatur als OCTOL-Prozeß Eingang gefunden (Hydrocarbon Process., Int. Ed. (1986) 65 (2, Sect. 1, 31-33). Die hieraus hergestellten tertiären Carbonsäuren können zu Vinylestern umgesetzt werden, die in Copolymerisaten eine besonders gute innere Weichmachung bewirken.

Die Verwendung von Butenoligomeren, insbesondere Dibuten und Tributen, zur Herstellung von Vinylestern weist eine Reihe von bemerkenswerten Vorteilen auf: Als Ausgangsprodukt können preiswerte technische C$_4$-Ströme wie Raffinat II, Raffinat III oder andere C$_4$-Olefine enthaltende Rohstoffe eingesetzt werden. Die im wesentlichen nur n-Butene als oligomerisierbare Komponenten enthaltende Schnitte wie Raffinat II oder III sind zur Herstellung von Comonomeren zur inneren Weichmachung besonders geeignet. Besonders überraschend und nicht zu erwarten war, daß die weichmachende Wirkung der erfindungsgemäß hergestellten Vinylester auf Basis der aus Dibuten erhaltenen tertiären C$_9$- Carbonsäuren bei Copolymerisation mit Vinylacetat oder Vinylchlorid ausgezeichnet und dem Standardprodukt auf Basis Tripropen mindestens ebenbürtig waren. Mit den entsprechend hergestellten Vinylestern auf Basis Tributen kann noch eine weitere Verbesserung erzielt werden.

Im erfindungsgemäßen Verfahren werden die Butenoligomere aus dem Oligomerisationsprodukt abgetrennt und jeweils zu den entsprechenden, um ein Kohlenstoffatom längeren Carbonsäuren umgesetzt. Dies kann durch säurekatalysierte Hydrocarboxylierung (KOCH-Reaktion) oder durch Hydroformylierung und anschließende Oxidation der so erhaltenen Oxoaldehyde erfolgen. Die KOCH-Synthese, wie in Falbe, New Synthesis with Carbon Monoxide, Springer Verlag, Berlin 1980, S. 372 ff, beschrieben, wird in der Praxis die bevorzugte Methode sein. Hier werden Olefine mit Kohlenmonoxid in Anwesenheit starker Säuren wie Schwefelsäure oder Borfluorid-Hydraten zu tertiären Carbonsäuren umgesetzt. Mit Cu+ als Cokatalysator erfolgt die Reaktion sogar schon unter Normaldruck und bei Umgebungstemperatur (Y. Souma, H. Sano; Bull. Chem. Soc. Jpn. 1974, 47, 1717).

Anschließend werden die so erhaltenen Carbonsäuren zu den entsprechenden Vinylestern umgesetzt. Dies kann beispielsweise durch Umsetzung der Carbonsäuren mit Acetylen, bevorzugt in Anwesenheit des Zinksalzes der zu vinylierenden Säure bei Normaldruck und 200-250 °C (z. B. gemäß Encyl, Polym. Sci. Eng. 17, S. 426-434) erfolgen.

Alternativ können die Vinylester durch Umesterung der Carbonsäuren mit weiteren Vinylestern wie z.B. Essigsäurevinylester oder Propionsäurevinylester (z.B. beschrieben in: Ullmann, 4. Auflage, Band 19, S. 368 ff) erhalten werden.

Die Weichmachereigenschaften der Vinylester werden, wie bereits ausgeführt, von dessen Verzweigungsgrad beeinflußt. Der Verzweigungsgrad der Vinylester kann wiederum durch den Verzweigungsgrad der Einsatzolefine beeinflußt werden. In besonderen Ausführungsformen der vorliegenden Erfindung können daher Dibutene mit einem Anteil von nicht mehr als 35, bevorzugt 25 Gew. % mehrfach verzweigter *Olefine, wie z. B. Dimethylhexen*, eingesetzt werden.

Die nach dem erfindungsgemäßen Verfahren hergestellten Vinylester können als Comonomere in Polymerisationsreaktionen, zum Beispiel in der Herstellung von Polyvinylacetat, verwendet werden, wo sie eine innere Weichmachung unter gleichzeitiger Erhöhung der Hydrolysestabilität bewirken. Copolymerisation mit Ethylen oder die Herstellung von Terpolymeren mit Acrylaten sind weitere Einsatzbeispiele für die Verwendung der erfindungsgemäß hergestellten Vinylester als Comonomere für die innere Weichmachung.

Die folgenden Beispiele sollen die Erfindung näher erläutern, ohne ihre Umfang zu begrenzen.

## Beispiel 1 bis 3

Im folgenden ist die typische Zusammensetzung von Dibutenen angegeben, die nach den drei verschiedenen Oligomerisierungsvarianten aus n-Butenen entstehen. Dabei ist die Produktzusammensetzung im wesentlichen nur von dem Oligomerisierungsverfahren abhängig. Eingesetzt werden kann beispielsweise Raffinat II oder Raffinat III oder andere n-buten-haltige Ströme, solange keine wesentlichen Mengen an verzweigten Butenen enthalten sind. Für die folgenden Beispiele wurde Raffinat III als Rohstoff eingesetzt.

Olefin-A     Dibuten, erhalten durch Oligomerisierung von Raffinat III an Montmorillonit ( Säurekatalyse)
Olefin-B     Dibuten, erhalten durch Oligomerisierung von Raffinat III nach dem DIMERSOL-Verfahren
Olefin-C     Dibuten, erhalten durch Oligomerisierung von Raffinat III nach dem OCTOL-Verfahren

| | Beispiel 1 | Beispiel 2 | Beispiel 3 |
| --- | --- | --- | --- |
| | Olefin A | Olefin B | Olefin C |
| n-Octen | ~ 0 % | ~ 6 % | ~13% |
| 3-Methyl-Hepten | ~ 5 % | ~ 59 % | ~62% |
| 3,4-Dimethyl-Hexen | ~ 70 % | ~ 34 % | ~24% |
| Sonstige $C_8$-Olefine | ~ 25 % | ~ 1 % | ~ 1 % |

Der Anteil an linearen und maximal einfach verzweigten Olefinen beträgt daher bei Olefin A ca. 5 %, bei Olefin B ca. 65 % und Olefin C ca. 75 %. Alle Angaben in Gew.-%.

## Beispiel 4 bis 6

Aus den Olefinen A, B und C wurden in Anlehnung an DE 23 39 947 tertiäre Carbonsäuren hergestellt. Als Katalysator diente ein Komplex aus Bor-Fluorid und Wasser, als Cokatalysator $Cu^+$. Die Umsetzungen erfolgten in einem Rührautoklaven in einem Temperaturbereich 20-35 °C und bei einem CO-Druck von 30 bar. Dabei wurde das Olefin über einen Zeitraum von 6 h gleichmäßig zudosiert. Durch Nachdosieren von CO wurde der Druck konstant gehalten. Die Umsetzung wurde beendet, sobald keine CO-Aufhahme mehr zu beobachten war.

Nach Abtrennung von der Katalysatorphase, Wasserwäsche und destillativer Aufarbeitung der rohen Carbonsäuren wurden Produkte folgender Zusammensetzung, jeweils über mehrere Ansätze, erhalten. (Werte in Massen-%).

| | Beispiel 4 | Beispiel 5 | Beispiel 6 |
| --- | --- | --- | --- |
| Isomere $C_9$-Carbonsäure | Säure A | Säure B | Säure C |
| 2,2-Dimethyl-Heptansäure | 0,5 % | 6,5 % | 7,4 % |
| 2-Methyl-2-Ethyl-Hexansäure | 3,7 % | 48,1 % | 55,2 % |

(fortgesetzt)

| Isomere C$_9$-Carbonsäure | Beispiel 4 | Beispiel 5 | Beispiel 6 |
| --- | --- | --- | --- |
| | Säure A | Säure B | Säure C |
| 2-Methyl-2-Propyl-Pentansäure | 0,5 % | 6,3 % | 7,2 % |
| 2,2-Diethyl-Pentansäure | 0,2 % | 3,0 % | 3,5 % |
| 2,2,5-Trimethyl-Hexansäure | 2,1 % | 1,1 % | 0,8 % |
| 2,2,4-Trimethyl-Hexansäure | 2,0 % | 1,0 % | 0,8 % |
| 2,4-Dimethyl-2-Ethyl-Pentansäure | 4,4 % | 2,2 % | 1,6 % |
| 2,2,3-Trimethyl-Hexansäure | 6,4 % | 3,2 % | 2,4 % |
| 2-Methyl-2-Isopropyl-Pentansäure | 13,7 % | 6,9 % | 5,1 % |
| 2,3-Dimethyl-2-Ethyl-Pentansäure | 37,5 % | 19,0 % | 13,8 % |
| 2-Ethyl-2-Isopropyl-Butansäure | 3,0 % | 1,5 % | 1,1 % |
| Unbekannte andere Säuren | 25,8 % | 1,2 % | 1,3 % |

**Beispiel 7 bis 9**

Die in Beispielen 4 bis 6 erhaltenen Gemische tertiärer Carbonsäuren wurden mit Acetylen bei Normaldruck bei einer Temperatur von 190-220 °C in Anwesenheit des Zinksalzes der jeweils umzusetzenden Säure nach der Gleichung umgesetzt.

$$R\text{-COOH} + HC{\equiv}CH \rightarrow R\text{-COO-CH=CH}_2$$

Die Reaktion erfolgt gemäß G. Hübner, Fette, Seifen, Anstrichmittel, 68 (4), S. 290-292 (1966).

Nach Destillation des Rohproduktes wurden Vinylester mit einer Reinheit von >= 99,8 % erhalten, die laut gaschromatografischer Untersuchung im wesentlichen einen den Carbonsäuren vergleichbaren oder sogar gleichen Verzweigungsgrad aufweisen. Die so erhaltenen Vinylester werden im Folgenden als Vinylester A (Basis Säure A, Beispiel 7), Vinylester B (Basis Säure B, Beispiel 8), Vinylester C (Basis Säure C, Beispiel 9), bezeichnet.

**Beispiel 10 bis 14**

Aus den Vinylestern gemäß Beispiel 7 bis 9 wurden Homopolymerisate nach Standardvorschrift hergestellt (Beispiele 12-14) und ihr Glaspunkt als Maß für die Eignung als Copolymere fiir innere Weichmachung ermittelt.

| Einsatzstoffe | |
| --- | --- |
| **Monomer** | **Gewichtsteile** |
| Vinylester der C$_9$-Carbonsäuren | 100,00 |
| **Wäßrige Phase** | |
| VE-Wasser | 70,00 |
| Anionisches Tensid, z. B. Marlon® A 390 (85 % aktive Substanz) | 0,03 |
| Nichtionisches Tensid, z. B. Marlophen® 820 (25 %ige Lösung) | 8,00 |
| Kaliumperoxodisulfat (K$_2$S$_2$O$_8$) | 0,10 |
| Kaliumcarbonat | 0,25 |
| Hydroxyethylcellulose, z. B. Natrosol 250 L (oder LR) | 2,00 |
| Essigsäure (100 %) | 0,20 |

(fortgesetzt)

| Einsatzstoffe | |
|---|---|
| **Monomer** | **Gewichtsteile** |
| **Initiatorlösung** | |
| Kaliumperoxodisulfat | 0,23 |
| VE-Wasser | 12,00 |

Durchführung

Die wäßrige Phase und etwa 10 % des Monomeren wurden unter Rühren auf 75 °C erwärmt. Nach 15 Minuten bei dieser Temperatur wurde das restliche Monomere und die Initiatorlösung in getrennten Strömen zudosiert. Die Zugabe des Monomeren erfolgte gleichmäßig in 120 Minuten und die der Initiatorlösung in 135 Minuten. Während der Dosierung wurde die Temperatur zwischen 75 und 80 °C gehalten. Nach weiteren 120 minütigen Rühren bei der gleichen Temperatur wurde der Ansatz auf Raumtemperatur abgekühlt. Aus der entstandenen Emulsion wurden, gegebenenfalls nach Filtration, Formkörper hergestellt, von denen durch Torsionsschwingungsanalyse (in Anlehnung an DIN 53445) die Glaspunkte bestimmt wurden.

Außerdem wurden zwei handelsübliche Vinylester, deren Glaspunkt bekannt ist, der gleichen Prozedur unterworfen, um die Vergleichbarkeit der Testprozedur sicherzustellen. Es handelte sich zum einen um einen Vinylester aus tertiären $C_{10}$-Säuren (VeoVa®10, Basis Tripropen, Vergleichsbeispiel 10), der weit verbreitet als innerer Weichmacher für beispielsweise Vinylacetat eingesetzt wird. Zum anderen wurde ein handelsüblicher Vinylester aus tertiären $C_9$-Säuren eingesetzt (VeoVa®9, Vergleichsbeispiel 11), also ein Vinylester mit der gleichen Summenformel jedoch anderem Verzweigungsgrad wie die gemäß den vorstehenden Beispielen hergestellten Vinylester.

Es wurden folgende Daten gemessen:

| | **Beispiel 10** | **Beispiel 11** | **Beispiel 12** | **Beispiel 13** | **Beispiel 14** |
|---|---|---|---|---|---|
| Einsatz | **(Vergleich)** | **(Vergleich)** | **Aus Säure A** | **Aus Säure B** | **Aus Säure C** |
| Glaspunkt | - 3 °C | ~ + 60 °C | ~ + 38 °C | + 1 °C | - 3 °C |

Vinylester A auf Basis von sauer katalysiert oligomerisiertem Buten ist somit ein hartmachendes Comonomeres. Vinylester B aus nach dem Dimersol-Verfähren oligomerisierten Buten ist schon ein weichmachendes Comonomeres. Mit Vinylester C, hergestellt aus Dibuten nach dem Octol-Verfahren, wird eine hervorragende weichmachende Wirkung erzielt, es ist dem Vergleichsprodukt auf Basis Tripropen (Vergleichbeispiel 10) ebenbürtig. Verwiesen sei auf den Vergleich von Beispiel 11 mit Beispiel 14. In beiden Fällen wurden Vinylester von tertiären Carbonsäuren mit 9 C-Atomen eingesetzt, also mit gleicher Summenformel. Mit dem erfindungsgemäßen hergestellten Vinylester Beispiel 14 wird hervorragende weichmachende Wirkung erzielt, während das Vergleichsbeispiel 11 ein ausgesprochen hartmachendes Comonomeres zeigt.

**Beispiel 15**

Raffinat III wurde nach dem Octol-Verfahren (siehe Beispiel 3) oligomerisiert. Aus dem Oligomerisat wurde das Tributen abgetrennt und durch Koch-Synthese (analog den Beispielen 4-6) ein $C_{13}$-Carbonsäuregemisch hergestellt. Dieses Gemisch wurde, wie in Beispiel 7-9 beschrieben, mit Acetylen zu den entsprechenden Vinylestern umgesetzt. Aus dem so erhaltenen Vinylestergemisch wurde analog der Vorschrift der Beispiele 10 bis 14 ein Homopolymerisat hergestellt; dessen Glaspunkt beträgt -13°C. Der Vinylester der $C_{13}$-Carbonsäure auf Basis von Tributen hat damit trotz des hohen Verzweigungsgrad eine ausgesprochen gute weichmachende Wirkung.

**Patentansprüche**

1. Verfahren zur Herstellung von Vinylestern aus Butenoligomeren
   **dadurch gekennzeichnet,**
   **daß**

a) Butene oligomerisiert,
b) die Butenoligomeren aus dem Oligomerisat abgetrennt,
c) die Butenoligomeren zu um ein Kohlenstoffatom längeren Carbonsäuren umgesetzt und
d) die Carbonsäuren zu den entsprechenden Vinylestern umgesetzt werden.

**2.** Verfahren nach Anspruch 1,
**dadurch gekennzeichnet,**
**daß** die Umsetzung der Butenoligomeren zu um ein Kohlenstoffatom längeren Carbonsäuren durch säurekatalysierte Hydrocarboxylierung erfolgt.

**3.** Verfahren nach Anspruch 1,
**dadurch gekennzeichnet,**
**daß** die Umsetzung der Butenoligomeren zu um ein Kohlenstoffatom längeren Carbonsäuren durch Hydroformylierung und anschließende Oxidation der so erhaltenen Aldehyde erfolgt.

**4.** Verfahren nach einem der Ansprüche 1 bis 3,
**dadurch gekennzeichnet,**
**daß** die Vinylester durch Umsetzen der Carbonsäuren mit Acetylen erhalten werden.

**5.** Verfahren nach Anspruch 4,
**dadurch gekennzeichnet,**
**daß** die Vinylester durch Umsetzen der Carbonsäuren mit Acetylen unter Anwesenheit eines Zinksalzes der Carbonsäure erhalten werden.

**6.** Verfahren nach einem der Ansprüche 1 bis 3,
**dadurch gekennzeichnet,**
**daß** die Vinylester durch Umesterung von weiteren Vinylestern mit den Carbonsäuren erhalten werden.

**7.** Verfahren nach Anspruch 6,
**dadurch gekennzeichnet,**
**daß** die weiteren Vinylester Essigsäurevinylester oder Propionsäurevinylester sind.

**8.** Verfahren nach einem der Ansprüche 1 bis 7,
**dadurch gekennzeichnet,**
**daß** Butenoligomeren Dibutene sind, die zu $C_9$-Carbonsäuren und den entsprechenden Vinylestern ungesetzt werden.

**9.** Verfahren nach einem der Ansprüche 1 bis 8,
**dadurch gekennzeichnet,**
**daß** Butenoligomeren Tributene sind, die zu $C_{13}$-Carbonsäuren und den entsprechenden Vinylestern umgesetzt werden.

**10.** Verfahren nach einem der Ansprüche 1 bis 8,
**dadurch gekennzeichnet,**
**daß** die Dibutene nicht mehr als 35 Gew. % mehrfach verzweigte Olefine enthalten.

**11.** Verfahren nach Anspruch 10,
**dadurch gekennzeichnet,**
**daß** die Dibutene nicht mehr als 25 Gew. % mehrfach verzweigte Olefine enthalten.

**Claims**

**1.** A process for preparing vinyl esters from butene oligomers, **characterized in that**,

a) butene is oligomerized,
b) the butene oligomers are separated off from the oligomerization product,
c) the butene oligomers are converted into carboxylic acids having one more carbon atom and

d) the carboxylic acids are converted into the corresponding vinyl esters.

**2.** A process according to claim 1,
   **characterized in that**
   the butene oligomers are converted into carboxylic acids having one more carbon atom by acid-catalysed hydrocarboxylation.

**3.** A process according to claim 1,
   **characterized in that**
   the butene oligomers are converted into carboxylic acids having one more carbon atom by hydroformylation and subsequent oxidation of the resulting aldehydes.

**4.** A process according to any one of claims 1 to 3,
   **characterized in that**
   the vinyl esters are obtained by reacting the carboxylic acids with acetylene.

**5.** A process according to claim 4,
   **characterized in that**
   the vinyl esters. are obtained by reacting the carboxylic acids with acetylene in the presence of a zinc salt of the carboxylic acid.

**6.** A process according to any one of claims 1 to 3,
   **characterized in that**
   the vinyl esters are obtained by transesterification of further vinyl esters with the carboxylic acids.

**7.** A process according to claim 6,
   **characterized in that**
   the further vinyl ester is vinyl acetate or vinyl propionate.

**8.** A process according to any one of claims 1 to 7,
   **characterized in that**
   the butene oligomers are dibutenes which are converted into $C_9$-carboxylic acids and the corresponding vinyl esters.

**9.** A process according to any one of claims 1 to 8,
   **characterized in that**
   the butene oligomers are tributenes which are converted into $C_{13}$-carboxylic acids and the corresponding vinyl esters.

**10.** A process according to any one of claims 1 to 8,
   **characterized in that**
   the dibutenes contain not more than 35% by weight of multiply branched olefins.

**11.** A process according to claim 10,
   **characterized in that**
   the dibutenes contain not more than 25% by weight of multiply branched olefins.

**Revendications**

**1.** Procédé de préparation d'esters vinyliques à partir d'oligomères de butène,
   **caractérisé en ce qu'**

   a) on oligomérise le butène,
   b) on sépare les oligomères de butène de l'oligornérisat,
   c) on convertit les oligomères de butène en acides carboxyliques plus longs d'un atome de carbone et
   d) on convertit les acides carboxyliques en esters vinyliques correspondants.

**2.** Procédé selon la revendication 1,
**caractérisé en ce que**
la conversion des oligomères de butène en acides carboxyliques plus longs d'un atome de carbone s'effectue par hydrocarboxylation catalysée par un acide.

**3.** Procédé selon la revendication 1,
**caractérisé en ce que**
la conversion des oligomères de butène en un acide carboxylique plus long d'un atome de carbone s'effectue par hydroformylation et oxydation consécutive de l'aldéhyde ainsi obtenu.

**4.** Procédé selon fine des revendications 1 à 3,
**caractérisé en ce que**
les esters vinyliques sont obtenus par réaction des acides carboxyliques avec l'acétylène

**5.** Procédé selon la revendication 4,
**caractérisé en ce que**
les esters vinyliques sont obtenus par réaction des acides carboxyliques avec l'acétylène en présence d'un sel de zinc de l'acide carboxylique.

**6.** Procédé selon l'une des revendications 1 à 3,
**caractérisé en ce que**
les esters vinyliques sont obtenus par réaction d'autres esters vinyliques avec les acides carboxyliques.

**7.** Procédé selon la revendication 6,
**caractérisé en ce que**
les autres esters vinyliques sont l'ester vinylique d'acide acétique ou l'ester vinylique d'acide propionique.

**8.** Procédé selon l'une des revendications 1 à 7,
**caractérisé en ce que**
les oligomères de butène sont des dibutènes qui sont convertis en acides carboxyliques en $C_9$ et en esters vinyliques correspondants.

**9.** Procédé selon l'une des revendications 1 à 8,
**caractérisé en ce que**
les oligomères de butène sont des tributènes qui sont convertis en acides carboxyliques en $C_{13}$ et en esters vinyliques correspondants.

**10.** Procédé selon l'une des revendications 1 à 8,
**caractérisé en ce que**
les dibutênes ne contiennent pas plus de 35 % d'oléfines plusieurs fois ramifiées.

**11.** Procédé selon la revendicadion 10,
**caractérisé en ce que** 1
les dibutenes ne contiennent pas plus de 25 % en poids d'oléfines plusieurs fois ramifiées.